Europäisches Patentamt

European Patent Office

Office européen des brevets

(19)

(11) Numéro de publication: **0 120 722**
A1

(12) # DEMANDE DE BREVET EUROPEEN

(21) Numéro de dépôt: **84400192.5**

(22) Date de dépôt: **27.01.84**

(51) Int. Cl.³: **A 61 K 7/00**
**A 61 K 9/50**

(30) Priorité: **08.02.83 FR 8301969**

(43) Date de publication de la demande:
**03.10.84 Bulletin 84/40**

(84) Etats contractants désignés:
**BE CH DE GB IT LI LU NL**

(71) Demandeur: **PARFUMS CHRISTIAN DIOR**
**30 avenue Hoche**
**F-75008 Paris(FR)**

(72) Inventeur: **Redziniak, Gérard**
**40 rue Jacques Duclos**
**F-78500 Sartrouville(FR)**

(72) Inventeur: **Meybeck, Alain**
**"Les Poissons" 20 Ter, Rue de Bezons**
**F-92400 Courbevoie(FR)**

(74) Mandataire: **Portal, Gérard et al,**
**Cabinet Z. Weinstein 20, Avenue de Friedland**
**F-75008 Paris(FR)**

(54) **Procédé pour stimuler la multiplication des cellules, composition cosmétique, pharmaceutique et composition complémentaire pour milieu de culture cellulaire appliquant ce procédé.**

(57) La présente invention concerne un procédé pour stimuler la multiplication des cellules par action de peptides ou polypeptides et des compositions cosmétiques, pharmaceutiques ou complémentaires pour culture cellulaire appliquant ce procédé.

Le procédé de l'invention consiste à encapsuler ou incorporer le ou les peptides ou polypeptides tels que des hydrolysats de proteines, par exemple un hydrolysat d'élastines ou un hydrolysat de collagène, ou un mélange de ceux-ci dans une phase lamellaire lipidique hydratée telle que, par exemple, des liposomes et à mettre en contact cette phase avec les cellules à traiter. Ainsi, selon ce procédé il est possible de diminuer la concentration en extraits biologiques dans les différentes compositions cosmétiques, pharmaceutiques ou complémentaires pour culture cellulaire sans diminuer leur activité sur les cellules.

La présente invention s'applique notamment dans les domaines de la culture des cellules et du traitement de la peau.

EP 0 120 722 A1

- 1 -

## Procédé pour stimuler la multiplication des cellules, composition cosmétique, pharmaceutique et composition complémentaire pour milieu de culture cellulaire appliquant ce procédé.

La présente invention concerne un procédé pour stimuler la multiplication des cellules, par exemple des fibroblastes.

Elle concerne également des compositions cosmétiques, pharmaceutiques ou complémentaires pour des milieux de culture cellulaire appliquant le procédé ci-dessus.

Pour stimuler la multiplication des cellules, notamment dans des cultures cellulaires, par exemple, pour la fabrication de vaccins, on utilise très fréquemment des extraits placentaires, des protéines provenant par exemple de peaux d'animaux ou de tendons, des peptides contenus dans le lait humain comme indiqué dans l'Article de Michäel Klagsbrun publié dans "Proc. Natl. Acad. Sci." USA, vol. 75 n° 10, page 5 057-5 061, Octobre 1978 sous le titre "Human Milk stimulates DNA synthesis and cellular proliferation in cultured fibroblasts", des sérums ou extraits de sérum tels que par exemple le sérum de veau, comme indiqué dans l'Article de C. DE LUCA et al. paru dans "Experimental Cell. Research 43, 98-106 (1966) sous le titre "The stimulation of Growth of mammalian cells in vitro by a peptide fraction from enzymatic digests of serum" et dans l'Article de Robert W. Pierson, Jr. et Howard M. Temin paru dans J. Cell. Physiol. 79.319-330 sous le titre "The partial purification from Calf serum of a fraction with multiplication-stimulating activity for chicken fibroblasts in cell culture and with non-suppressible insulin-like activity".

On sait également, par exemple, à partir des brevets français n° 2.353.282, 2.472.385 et 2.380.294 qu'il est possible d'utiliser des composants peptidiques, tels que le collagène, l'élastine dans des compositions cosmétiques pour par exemple la régénérescence de la peau.

Toutefois, l'utilisation de substances biologiques, comme les sérums, les extraits placentaires, les extraits produits par la macération de tissus provoque parfois des phénomènes d'intolérance quand elles sont appliquées, même à l'état dilué, sur la peau. Par ailleurs, il est parfois difficile d'incorporer ces substances biologiques ou les composants peptidiques dans des compositions cosmétiques, pharmaceutiques ou autres, sans diminuer l'activité de ces substances sur les cellules.

La présente invention a notamment pour but de remédier à ces inconvénients en proposant un procédé permettant d'appliquer des substances peptidiques sur la peau, de les administrer sous forme de médicaments, ou de les additionner dans des milieux de culture cellulaire pour stimuler la multiplication des cellules, en supprimant sensiblement ou totalement les risques d'intolérance par rapport à la peau ou au milieu de culture cellulaire ou à l'organisme humain, et en augmentant sensiblement l'activité de ces substances peptidiques sur la cellule notamment sur les fibroblastes. Ainsi, le procédé de l'invention permet de remplacer, notamment dans les milieux de culture cellulaire, partiellement ou totalement les sérums ou extraits placentaires ou analogues.

A cet effet, l'invention propose un procédé pour stimuler la multiplication des cellules, notamment des fibroblastes, par action de peptides ou polypeptides sur lesdites cellules, caractérisé en ce qu'il consiste à incorporer ou encapsuler le ou lesdits peptides ou polypeptides ou un mélange de ceux-ci dans une phase lamellaire lipidique hydratée telle que, par exemple, des liposomes, et à mettre en contact lesdites particules avec les cellules à traiter.

Avantageusement le ou les peptides ou polypeptides précités sont obtenus par hydrolyse de protéines ou de macromolécules de structure des tissus, notamment l'élastine, la fibroïne, la kératine, le collagène, la myosine, l'actine, la tubuline, la fibrine ou analogue.

Selon une autre caractéristique de l'invention, le peptide ou polypeptide précité est un hydrolysat d'élastine ou de collagène de poids moléculaire compris entre environ 1 000 et 75 000.

De préférence, les constituants lipidiques formant la phase lamellaire lipidique hydratée précitée sont choisis parmi les composés appartenant à la famille des glycolipides, phospholipides ou phospho-aminolipides et avantageusement sont la lécithine de soja ou d'oeuf.

Selon une nouvelle caractéristique de l'invention, des substances à caractère hydropho e tel que l'ubiquinone, des stérols, par exemple le cholestérol , le lanostérol, le stigmastérol, le sitostérol, sont incorporées aux couches de la phase lamellaire lipidique hydratée précitée.

L'invention a également pour objet une composition cosmétique pour la revitalisation de la peau, caractérisée en ce qu'elle applique le procédé décrit ci-dessus. Cette composition cosmétique comprend la phase lamellaire lipidique hydratée dans laquelle sont encapsulés ou incorporés le ou les peptides ou polypeptides, mélangée à au moins un excipient convenable pour obtenir par exemple, une lotion, une crème, un baume, un gel ; la concentration pondérale en lipides constituant la phase lamellaire lipidiques dans ladite composition est comprise entre environ 0,01 % et environ 80 %. Avantageusement, le peptide ou le polypeptide incorporé ou encapsulé dans les couches de la phase lamellaire lipidique hydratée précitée est un hydrolysat d'élastine ou un hydrolysat de collagène.

L'invention a également pour objet une composition pharmaceutique pour notamment favoriser la cicatrisation de plaies, brûlures ou analogues, appliquant le procédé décrit ci-dessus et contenant une quantité thérapeutiquement efficace de la phase lamellaire lipidique hydratée précitée dans laquelle sont incorporés ou encapsulés le ou les peptides ou polypeptides précités, mélangés avec un excipient physiologiquement compatible.

L'invention a également pour objet une composition complémentaire pour milieu de culture cellulaire utilisé par exemple pour la fabrication de vaccins, de produits chimiques par action de micro-organismes, ou pour réaliser des tests de toxicité. Cette composition complémentaire comprend une quantité efficace de phase lamellaire lipidique hydratée dans laquelle sont encapsulés ou incorporés le ou les peptides ou polypeptides précités, mélangés éventuellement avec un excipient convenable pour la culture cellulaire, par exemple un milieu minimum d'Eagle (M.E.M.).

De préférence, la concentration pondérale en lipides constituant la phase lamellaire lipidique hydratée, dans ladite composition complémentaire est comprise entre environ 0,01 % et environ 80 %. Avantageusement cette concentration est comprise entre environ 0,01 % et environ 20 %.

Ainsi, selon l'invention les peptides ou polypeptides par exemple obtenus par hydrolyse de protéines, sont incorporés ou encapsulés dans des phases lamellaires lipidiques hydratées pour former notamment des liposomes, pour faciliter l'application des peptides ou polypeptides sur les cellules et notamment la pénétration de ces substances dans la peau. Donc le procédé de l'invention permet d'améliorer de manière sensible l'efficacité des peptides ou polypeptides sur les fibroblastes et, en conséquence, augmente les effets de revitalisation de la peau des compositions cosmétiques contenant ces liposomes. De plus, comme les constituants lipidiques formant les liposomes selon l'invention , sont facilement acceptés par la peau humaine, les risques d'intolérance sont diminués ou même supprimés.

Par ailleurs, le procédé de l'invention permet de remplacer partiellement ou totalement les substances biologiques utilisées dans les compositions cosmétiques, pharmaceutiques ou complémentaires pour milieu de culture cellulaire, telles que par exemple les sérums, les extraits placentaires, les extraits embryonnaires ou analogues, qui sont d'un coût de revient relativement élevé et, par ailleurs contiennent de nombreuses substances ne stimulant pas la multiplication des cellules.

L'invention sera mieux illustrée et d'autres caractéristiques, détails et avantages de celle-ci apparaîtront plus clairement au vu des exemples suivants non limitatifs, et donnés uniquement à titre d'illustration de la présente invention.

Les concentrations indiquées dans les exemples suivants sont des concentrations pondérales, la concentration en phase phospholipidique représente la concentration pondérale en lipides constituant cette phase phospholipidique, dans la composition.

Exemple 1 :
Préparation d'une lotion cosmétique ayant un effet adoucissant pour la peau du visage.
La composition de cette lotion cosmétique est :
- phase phospholipidique
  contenant 10 % de cholestérol :         1 %
- polypeptides d'élastine (hydrolysat)    1 %
- excipients aqueux stabilisés par des
  conservateurs                     q.s.p. 100

La phase phospholipidique contenant le cholestérol est préparée selon un procédé convenable et notamment selon le procédé d'atomisation décrit dans la demande de brevet européen n° 83400281.8 du 9 février 1983. 20 grammes de poudre ainsi obtenue sont dispersés dans 100 grammes "d'eau stabilisée" contenant 20 grammes de polypeptides d'élastine, puis on homogénéise cette dispersion par, par exemple, le procédé d'homogénéisation décrit dans la demande de brevet européen n° 83401954.9 du 6 octobre 1983 pour obtenir une suspension de particules ayant des dimensions désirées. A cette suspension, on ajoute la quantité nécessaire d'excipients aqueux stabilisés pour obtenir les concentrations indiquées ci-dessus.

La lotion ainsi obtenue est appliquée quotidiennement par exemple après le nettoyage matinal de la peau. On obtient une peau très douce sur laquelle on peut appliquer facilement un maquillage.

Exemple 2 :
Préparation d'une composition cosmétique utilisée pour les soins de beauté de la peau du visage.

Composition :
- phase phospholipidique à
  10 % de sitostérol                              5 %
- polypeptides de collagène (hydrolysat)   1 %
- polypeptides d'élastine (hydrolysat)     1 %
- excipients aqueux stabilisés par
  des conservateurs                         q.s.p. 100

La préparation de la phase phospholipidique, l'encapsulation du polypeptide de collagène et polypeptide d'élastine dans la phase phospholipidique, et l'homogénéisation de la dispersion ainsi obtenue sont effectuées comme dans l'exemple 1.

L'application répétée pendant 8 jours de ce produit sur la peau du visage permet d'obtenir une peau plus belle, plus souple et plus tendue.

Exemple 3 :
Préparation d'un gel cosmétique pour la peau du visage:

Composition :
- phase phospholipidique à
  10 % de lanostérol                              2 %
- hydrolysat de collagène                         1 %
- eau "stabilisée" par des
  conservateurs et excipients
  pour la préparation d'un gel              q.s.p. 100%

La préparation de la phase phospholipidique contenant du lanostérol et l'encapsulation de l'hydrolysat de collagène sont réalisées selon le procédé décrit dans l'exemple 1.

Après dispersion de la phase lamellaire lipidique hydratée ainsi obtenue dans de l'eau "stabilisée" et homogénéisation selon le procédé décrit dans l'exemple 1, on ajoute les excipients pour la préparation d'un gel, à savoir un polymère carboxyvinylique et la triéthanolamine.

L'application quotidienne de ce gel sur la peau permet d'hydrater celle-ci et la rend plus douce et plus souple.

Exemple 4 :
Préparation d'un masque de traitement de soins de beauté de la peau du visage.
  Composition :
  - phase phospholipidique à
    10 % de sitostérol                     20 %
  - hydrolysat d'élastine                   2 %
  - excipients aqueux stabilisés
    avec des conservateurs            q.s.p. 100

La préparation de la phase phospholipidique, l'encapsulation de l'hydrolysat d'élastine et l'homogénéisation de la dispersion de la phase lamellaire lipidique hydratée sont réalisées selon les procédés de l'exemple 1.

Le produit ainsi obtenu est appliqué sur la peau du visage et laissé pendant au moins 1 heure, et avantageusement pendant une nuit.

La peau est ensuite rincée par de l'eau fraîche ou avec une lotion puis séchée par petits tapotements avec une serviette. Ce traitement rend la peau plus ferme et plus tendue.

Exemple 5 :

Préparation d'une lotion de beauté pour la peau du corps.

Composition :
- phase phospholipidique contenant 5 % de    2 % sitostérol
- polypeptides de fibroïne de soie    1 %
- excipients aqueux "stabilisés" avec des conservateurs    q.s.p.100

La préparation de la phase phospholipidique, l'encapsulation des polypeptides de fibroïne de soie, et l'homogénéisation de la dispersion obtenue sont réalisés selon les procédés de l'exemple 1.

La lotion ainsi obtenue est appliquée sur la peau du corps après chaque bain ou douche. Ce traitement rend la peau plus souple et plus douce.

Exemple 6 :

Préparation d'une composition cosmétique de soins de beauté des ongles.

Composition :
- phase phospholipidique contenant 7 % de lanostérol    80 %
- hydrolysat de Kératine (polypeptides)    1 %
- excipients    q.s.p.100

Le mélange de constituants lipidiques est obtenu par le procédé d'atomisation de l'exemple 1. La quantité nécessaire d'excipients contenant, en solution, l'hydrolysat de Kératine est préparée puis mélangée au mélange pulvérulent lipidique atomisé. Ce mélange est homogénéisé au moyen d'un broyeur à rouleaux. On obtient ainsi une phase lamellaire lipidique peu hydratée.

L'application de cette composition sur les ongles les rend moins cassants.

Exemple 7 :
Préparation d'une composition pharmaceutique activant la cicatrisation des plaies.

Composition :
- phase phospholipidique          2 %
- hydrolysat d'élastine           3 %
- carraghénane                    0,8 %
- glycérol                        5 %
- excipients                      q.s.p. 100

La phase phospholipidique est préparée selon le procédé décrit dans la demande de brevet européen n° 83400281.8 du 9 février 1983. La poudre obtenue est dispersée dans une solution d'hydrolysat d'élastine pour obtenir ainsi des phases lamellaires lipidiques hydratées.

La dispersion ainsi obtenue est homogénéisée, par exemple selon le procédé décrit dans la demande de brevet européen n° 83401954.9 du 6 octobre 1983.

Ces phases lamellaires lipidiques hydratées sont mises en suspension dans de l'eau "stabilisée" contenant le carraghénane et le glycérol.

La suspension obtenue est mélangée avec un excipient physiologiquement compatible pour former un baume qui sera appliqué pendant 8 jours après la fermeture d'une plaie. Ce produit accélère la cicatrisation de cette plaie.

Exemple 8 :

Préparation d'une composition pharmaceutique pour activer la cicatrisation des plaies.

Composition. :

- phase phospholipidique à
  5 % de lanostérol                         20 %
- hydrolysat d'élastine                       2 %
- excipients aqueux "stabilisés"
  par des conservateurs                q.s.p. 100

La préparation de la phase phospholipidique contenant du lanostérol, l'encapsulation de l'élastine et l'homogénéisation de la suspension de la phase lamellaire lipidique hydratée ainsi obtenue ou de liposomes sont réalisées selon les procédés de l'exemple 1.

Cette suspension appliquée quotidiennement après la fermeture d'une plaie accélère la cicatrisation de celle-ci.

Exemple 9 :

Préparation d'une pommade pour peau gercée.

Composition :

- phase phospholipidique à
  5 % de lanostérol                         60 %
- hydrolysat de collagène                     1 %
- excipients gras                       q.s.p.100

La préparation de la phase phospholipidique peu hydratée est obtenue selon le procédé décrit dans l'exemple 6, l'hydrolysat de collagène étant ajouté à l'excipient gras.

La pommade obtenue est appliquée quotidiennement, avant et après l'exposition de la peau aux basses températures.

-12-

Exemple 10 :

Cet exemple concerne des compositions complémentaires pour des milieux de culture cellulaire et illustre les résultats obtenus par le procédé de l'invention sur la vitesse de multiplication des cellules en comparaison aux produits complémentaires pour culture cellulaire connus.

Pour cela, on prépare des compositions A, B contenant un hydrolysat d'élastine ou un hydrolysat de collagène non encapsulé dans une phase lamellaire lipidique hydratée, et des compositions complémentaires C, D conformes au procédé décrit dans l'exemple 1.

Composition A.
- hydrolysat d'élastine (PM $\simeq$ 4000)          1 %
- soluté physiologique                              q.s.p.100

Composition B.
- hydrolysat de collagène (PM $\simeq$ 4000)        1 %
- soluté physiologique                              q.s.p.100

Composition C.
- phase phospholipidique                            1 %
- hydrolysat d'élastine (PM $\simeq$ 4000)          1 %
- soluté physiologique                              q.s.p.100

Composition D.
- phase phospholipidique                            1 %
- hydrolysat de collagène (PM $\simeq$ 4000)        1 %
- soluté physiologique                              q.s.p.100

Les compositions C, D sont fabriquées selon les procédés décrits à l'exemple 1. Elles sont filtrées, de même que les compositions A et B à travers un filtre ayant des pores de 0,22 nanomètres.

On effectue des tests de comparaison sur une culture de fibroblastes de souris. Cette culture est réalisée dans des boîtes de Pétri dans un milieu M.E.M. contenant 100 microgrammes par millilitre de streptomycine et
100 UI par millilitre de pénicilline.

Pour déterminer l'activité des compositions complémentaires A, B, C, D, on ajoute dans une première boîte
de Petri 5 % de sérum de veau et dans une seconde
boîte 10 % de sérum de veau. On détermine le nombre de
cellules produites dans chacune de ces deux boîtes
témoins après un temps déterminé :

$N_t^{10}$ représente le nombre de cellules produites dans
la boîte de Petri témoin contenant 10 % de sérum de
veau.

$N_t^5$ représente le nombre de cellules produites
dans la boîte de Petri contenant 5 % de sérum de veau.

Pour déterminer l'activité des compositions complémentaires A, B, C, D, on remplace une certaine quantité
du milieu de culture contenant 10 % de sérum de veau
contenu dans une boîte de Petri, par une quantité
équivalente d'un milieu frais de culture contenant
5 % de sérum de veau et une composition complémentaire
A, B, C ou D, avec une concentration en composition
égale à 100 microgrammes par millilitre de milieux  de
culture  à 5 % de sérum. On remplace également dans
les boîtes de Petri témoins la même quantité de milieu
de culture par un milieu frais contenant respectivement
5 % de sérum de veau pour la boîte de Petri témoin à
5 % et 10 % de sérum de veau pour la boîte de Pétri
témoin à 10 % ; mais ce milieu frais ne contient pas
de composition complémentaire A, B, C ou D.

Dans le présent  exemple, 2 millilitres de milieu
M.E.M. contenant soit 5 % de sérum de veau, soit 10 %
de sérum de veau sont ensemencés avec $2 \times 10^5$ cellules.
Après un jour, on remplace dans chaque boîte de Petri 2
millilitres du milieu de culture existant par 2 millilitres de milieu frais contenant ou non une composition complémentaire A, B, C ou D, et bien entendu soit 5 %, soit 10 % de sérum de veau.

Après 3 jours de culture, et un traitement à la trypsine pour décoller les cellules, on dénombre les cellules produites. Le nombre de cellules produites dans les boîtes de Petri contenant une composition complémentaire A, B, C ou D sont exprimés par $N_e^{Co}$.

L'activité des compositions complémentaires A, B, C, D est donné par la formule suivante :

$$\text{activité} = \frac{N_e^{Co} - N_t^5}{N_t^{10} - N_t^5} \times 100$$

Les résultats obtenus sont indiqués dans le tableau 1 ci-dessous.

Tableau 1.

| composition complémentaire | concentration finale en composition complémentaire $\mu$g par ml de milieu de culture à 5 % en sérum de veau | ACTIVITE |
|---|---|---|
| hydrolysat d'élastine (A) | 100 | 57 % |
| liposomes + hydrolysat d'élastine (C) | 100 | 108 % |
| hydrolysat de collagène (B) | 100 | 41 % |
| liposomes + hydrolysat de collagène (D) | 100 | 87 % |

Il apparaît clairement à partir de ces résultats que la caractéristique consistant à encapsuler un peptide ou un polypeptide dans une phase lamellaire lipidique hydratée avant son addition dans un milieu de culture augmente de manière importante et significative son action sur la multiplication des cellules. Pour les deux peptides étudiés, cette activité a été environ doublée.

En conséquence, le procédé de l'invention permet de remplacer partiellement ou totalement les extraits biologiques utilisés actuellement pour stimuler la multiplication des cellules par des produits moins coûteux et plus purs permettant d'incorporer dans ces cultures des composants connus à des concentrations connues. D'autre part, l'addition d'extraits biologiques ajoute de manière inhérente un nombre relativement important de substances à des concentrations plus ou moins fortes, substances qui n'ont aucune influence sur la multiplication des cellules qui peuvent même diminuer l'effet des substances efficaces contenues dans lesdits extraits biologiques, ce qui est minimisé ou évité par la présente invention.

Revendications.

1. Procédé pour stimuler la multiplication des cellules, notamment des fibroblastes, par action de peptides ou polypeptides, caractérisé en ce qu'il consiste à encapsuler ou incorporer le ou lesdits peptides ou polypeptides ou un mélange de ceux-ci dans une phase lamellaire lipidique hydratée telle que, par exemple, les liposomes, et à mettre en contact ladite phase avec les cellules à traiter.

2. Procédé selon la revendication 1, caractérisé en ce que le ou les peptides ou polypeptides précités sont obtenus par hydrolyse de protéines, ou de macromolécules de structure de tissus, notamment l'élastine, la fibroïne, la kératine, le collagène, la myosine, l'actine, la tubuline, la fibrine.

3. Procédé selon la revendication 1 ou 2, caractérisé en ce que le peptide ou le polypeptide précité est un hydrolysat d'élastine ou hydrolysat de collagène, de poids moléculaire compris entre environ 1 000 et 75 000.

4. Procédé selon l'une des revendications 1 à 3, caractérisé en ce que les constituants lipidiques formant la phase lamellaire lipidique hydratée précitée sont choisis parmi les composés appartenant à la famille des glycolipides, phospholipides, ou phospho-aminolipides, et de préférence sont la lécithine de soja ou d'oeuf.

5. Procédé selon l'une des revendications précédentes, caractérisé en ce que des substances à caractère hydrophobe telles que l'ubiquinone, des stérols, par exemple, le cholestérol, le lanostérol, le stigmastérol, le sitostérol sont incorporées aux couches de la phase lamellaire lipidique hydratée précitée.

6. Composition cosmétique pour la revitalisation de la peau, caractérisée en ce qu'elle applique le procédé selon l'une des revendications 1 à 5.

7. Composition cosmétique selon la revendication 6, caractérisée en ce que la phase lamellaire lipidique hydratée précitée contenant le ou les peptides ou les polypeptides précités est mélangée à au moins un excipient convenable pour obtenir, par exemple, une lotion, une crème, un baume, un gel, la concentration pondérale en lipides constituant ladite phase lamellaire lipidique dans ladite composition est comprise entre environ 0,01 % et environ 80 %.

8. Composition cosmétique selon la revendication 7, caractérisée en ce que le peptide ou le polypeptide incorporé ou encapsulé dans la phase lamellaire lipidique hydratée est un hydrolysat d'élastine ou un hydrolysat de collagène.

9. Composition pharmaceutique pour notamment favoriser la cicatrisation de plaies ou de brûlures, caractérisée en ce qu'elle applique le procédé selon l'une des revendications 1 à 5.

10. Composition pharmaceutique selon la revendication 9, caractérisée en ce que la concentration pondérale en lipides constituant ladite phase lamellaire lipidique dans ladite composition est comprise entre environ 0,01 % et environ 80 %.

11. Composition complémentaire à des milieux de culture cellulaire pour favoriser la multiplication des cellules, caractérisée en ce qu'elle applique le procédé selon l'une des revendications 1 à 5 .

12. Composition selon la revendication 11, caractérisée en ce que la concentration pondérale en lipides constituant ladite phase lamellaire lipidique dans ladite composition complémentaire est comprise entre environ 0,01 % et environ 80 % ; de préférence entre environ 0,01 % et environ 20 %.

13. Composition selon la revendication 11 ou 12, caractérisée en ce que le peptide ou polypeptide précité encapsulé ou incorporé dans la phase lamellaire lipidique hydratée est un hydrolysat de collagène ou un hydrolysat d'élastine.

Office européen
des brevets

**RAPPORT DE RECHERCHE EUROPEENNE**

**0120722**
Numéro de la demande

EP  84  40  0192

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| X,Y | DIE PHARMAZIE, vol. 34, no. 5/6, mai/juin 1979, pages 350-351, DD J. AXT et al.: "Liposomen als potentielle Peptidwirkstoff-Carrier - Untersuchungen mit Insulin als Modell - " * Article entier * | 1,4,5 | A 61 K  7/00 A 61 K  9/50 |
| X,Y | FR-A-2 399 242  (BATTELLE MEMORIAL INSTITUTE) *  Page  11,  ligne 29 - page 12, ligne  20; revendications 1,5,6,8 * | 1,4-7 | |
| D,Y | FR-A-2 472 385  (SOCIETE SEDERMA) *  Page  1,  ligne  31  - page 2, ligne  40;  page  5,  lignes 8-15; revendications * | 1-3,6-8,11 | DOMAINES TECHNIQUES RECHERCHES (Int. Cl. ³) |
| D,Y | CHEMICAL ABSTRACTS, vol. 77, no. 17, 23 octobre 1972, page 49, no. 109662h, Columbus, Ohio, US R.W.  PIERSON Jr. et al.:  "Partial purification from calf serum of  a  fraction  with multiplication-stimulating activity  for  chicken  fibroblasts in cell  culture  and  with non-suppressible  insulin-like activity" & J.  CELL.  PHYSIOL. 1972, 79(3), 319-29 * Résumé * | 1,6,11 | A 61 K  7/00 A 61 K  9/00 |

-/-

Le présent rapport de recherche a été établi pour toutes les revendications

| Lieu de la recherche LA HAYE | Date d'achèvement de la recherche 04-06-1984 | Examinateur BERTE M.J. |
|---|---|---|

Office européen
des brevets

**RAPPORT DE RECHERCHE EUROPEENNE**

EP 84 40 0192

| | DOCUMENTS CONSIDERES COMME PERTINENTS | | Page 2 |
|---|---|---|---|
| Catégorie | Citation du document avec indication, en cas de besoin, des parties pertinentes | Revendication concernée | CLASSEMENT DE LA DEMANDE (Int. Cl. ³) |
| D,Y | FR-A-2 353 282 (ETABLISSEMENTS D'INVENTIONS ET D'APPLICATIONS) * Page 1 - page 4, ligne 3; page 7, alinéa 1; revendications * | 1-3,6-8,11 | |
| Y | SEIFEN-ÖLE-FETTE-WACHSE, vol. 107, no. 13, auùt 1981, page 376, Augsburg, DE "Proteine in der Kosmetik" * Résumé en entier * | 2,3,6-8,11 | |
| Y | HOUSEHOLD & PERSONAL PRODUCTS INDUSTRY, vol. 17, no. 1, janvier 1980, page 17, Denville, US "Vidal Sassoon skin care" * Résumé en entier * | 2,3,6-8,11 | |
| | | | DOMAINES TECHNIQUES RECHERCHES (Int. Cl. ³) |

Le présent rapport de recherche a été établi pour toutes les revendications

| Lieu de la recherche LA HAYE | Date d'achèvement de la recherche 04-06-1984 | Examinateur BERTE M.J. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82